# EUROPEAN PATENT SPECIFICATION

(11) **EP 1 862 194 B1**
(45) Date of publication and mention of the grant of the patent: **08.04.2009**
(21) Application number: 06018265.6
(22) Date of filing: 31.08.2006
(51) Int. Cl.: A61N 1/32, A61N 1/20

(54) **Electric therapeutic appliance for improving immunity**
Elektrotherapiegerät zur Verbesserung der Immunfunktionen
Appareil thérapeutique à potentiel électrique destiné à améliorer l'immunité

(30) Priority: 31.05.2006 JP 2006152099
(43) Date of publication of application: 05.12.2007
(73) Proprietor: Serumi Medical Instruments Co., Ltd., Sakaide-city Kagawa 762-0025 (JP)
(72) Inventor: Horiguchi, Noboru, Ayauta-gun Kagawa 769-0213 (JP); Horiguchi, Hiroshi, Sakaide-city Kagawa 762-0025 (JP)
(74) Representative: Leinweber & Zimmermann

(56) References cited:
- EP-A- 1 591 141
- GB-A- 1 427 236
- GB-A- 2 181 059
- US-A- 5 690 692

## Description

### BACKGROUND OF THE INVENTION

### Field of the Invention

The present invention relates to an improvement in an electrotherapeutic device effective in improving immunity by generating negative or reducing ions in a patient body.

### Description of the Related Art

In recent years, the cases demonstrating that negative ions or reducing ions are good for a human body have been accumulated. Not only the efficacy for health but also the treatment effect has been clarified. In particular, the immunity and natural healing power a human being possesses by birth are improved by utilizing the effect of the reducing ions, whereby the effective treatment is expected. An electrotherapeutic device having the function to generate the reducing ions has been developed.

Patent Document 1 listed below discloses an electric potential therapeutic instrument having the function for discharging reducing ions. The electric potential therapeutic instrument disclosed in Patent Document 1 has a treatment electrode, which is used for electric potential therapy, realized with a porous pad. The treatment electrode includes a needle electrode and a curving plate electrode opposed to the needle electrode so as to induce corona discharge. When the potential at the electrode pad is higher than a ground potential, reducing ions generated in the treatment electrode are supplied to a patient through the patient's skin via the numerous holes of the pad, as soon as electric potential therapy is initiated.

The electric potential therapeutic instrument is requested to generate a large number of reducing ions. However, even if a large number of reducing ions are generated by corona discharge, a treatment effect is not always provided.

In order to have an effect of reducing ions on a living body, it has significant meaning how effectively electrons or charges generated by the electric potential therapeutic instrument are taken into the living body.

For application of reducing ions to a living body, the property of generated reducing ions should be taken into consideration. Namely, the reducing ions should exhibit the same mobility as negative cluster ions that are generated in the air as a natural phenomenon. Herein, the mobility refers to a velocity at which ions move under the influence of an electric field or a degree of readiness with which reducing ions make motion. When the mobility of ions is high, the ions are likely to chemically react to any other substances, and reduction or alkalization of the ions is likely to occur even in the air. Generally, when the reducing ions that consist of cluster ions exhibit high mobility, they are thought to effectively suppress acidification and oxidization in a living body.

For artificial generation of reducing ions exhibiting high mobility, it is more effective that charges are accumulated to some extent and then released at a stroke than that electrons or charges are released constantly. Owing to the intermittent discharge method, even after discharge is finished, the reducing ions generated in the air remain for a longer period of time and the reducing ions are observed to have a longer life. Moreover, when a mechanism that is expected to effectively generate the reducing ions in the air is used to apply an induced electric field directly to a living body, the effect of reducing ions are known to be produced in a humor so as to assist medical treatment or improve physical constitution.

The efficacy of reducing ions is described in Patent Documents 2 and 3 listed below which are filed previously by the present applicant. The invention disclosed in Patent Document 2 is an electric potential therapeutic instrument comprising a waveform shaper realized with moisturized pumice blocks filled in. The invention disclosed in Patent Document 3 is an electric potential therapeutic instrument comprising a waveform shaper realized with inorganic insulator powder and a predetermined amount of moisture filled in.

The waveform shaper serves as an electric element that feeds current having nearly constant cycles when a high voltage is applied between both the terminals thereof. However, even when a short circuit current occurs caused by dielectric breakdown, the large resistance limits the flowing current between the electrodes.

The electrodes connected to the waveform shaper are disposed to sandwich a hand whose bloodstream is gushing. When a current of the specific waveform is applied, immunocompetent cells in bloodstream increase and a natural healing power is intensified. This phenomenon results in an improved treatment effect.

A patient is applied electricity not with a constant voltage but with a waveform of a predetermined potential having fine ripples superimposed thereon. The ripple waveform is important in treatment. The disclosed therapeutic device should be called an electrotherapeutic device.

When an attempt is made to exert a high treatment effect using the electrotherapeutic device, a current having an appropriate pattern should be adopted.

The disclosed electrotherapeutic device can regulate a treatment current by controlling an output voltage. However, during one treatment cycle of several tens of minutes, a patient treatment environment may vary, e.g. patient's sweating may decrease the resistance between the electrodes. Therefore, although the same voltage is applied, the current varies. Unless a paramedical personnel frequently regulates the voltage, the target current cannot be retained.
[Patent Document 1] Japanese Unexamined Patent Publication No. 2001-309987
[Patent Document 2] Japanese Unexamined Patent Publication No. 2005-046771
[Patent Document 3] Japanese Unexamined Patent Publication No. 2005-296441

An electrotherapeutic device according to the preamble of claim 1 is known from EP 1 591 141.

### SUMMARY OF THE INVENTION

An object of the present invention is to provide an electrotherapeutic device that comprises a special waveform shaper which reshapes a voltage waveform to be applied to the electrodes so that negative ions or reducing ions will be effectively generated, and that is easy to operate during treatment.

In order to accomplish the above objective, an electrotherapeutic device in accordance with the present invention arranges a waveform shaper filled with pumice blocks or inorganic insulator powder and a predetermined amount of moisture in series between a direct current high negative voltage generator and an output terminal, and supplies an output current fed from the high voltage power supply to the output terminal via the waveform shaper. The electrotherapeutic device comprises a current sensor for measuring a treatment current, and a program controller for varying an output voltage according to a predetermined program. The program controller applies a certain predetermined voltage until the predetermined acclimatization time has elapsed from the start of the treatment. Once the acclimatization time has elapsed, if the treatment current falls outside a predetermined regulative current range, the program controller regulates the input voltage of the waveform shaper so that the treatment current will gradually approach the regulative current range. After the treatment current reaches the regulative current range, the program controller regulates the input voltage of the waveform shaper so that the treatment current will gradually vary within the regulative current range. Thus, the program controller regulates the output voltage to be applied during a succeeding treatment period.

The reducing ions can act more effectively on the human body by repeatedly accumulating the charges in the electrodes and releasing them at a stroke, compared with providing charges through a constant electric field by directly applying the negative potential fed from the high voltage circuit.

The electrotherapeutic device in accordance with the present invention is an improvement upon the electro-potential therapeutic instrument as disclosed in Patent Document 2 or 3, that includes a waveform shaper of simple structure which generates reducing ions more effectively.

The waveform shaper employed in the present invention is a slightly conductive capacitor having a pair of electrode plates filled with pumice blocks or inorganic insulator powder and a predetermined amount of moisture, and sealed. In the thus structured waveform shaper, when a high voltage is applied between both the electrode plates, electric charges are accumulated according to the electrostatic capacity. However, if the voltage exceeds the withstand voltage, so-called dielectric breakdown occurs. Consequently, the electric charges move between the electrode plates, and a current flows. When the discharge causes the voltage reduction between the electrode plates, the property of insulating recovers and charging starts again. When the potential difference between the electrodes reaches a certain voltage, dielectric breakdown recurs and a current flows.

The waveform shaper serves as an electric element that feeds the current in a nearly constant cycle when a high voltage is applied between both the terminals thereof. However, even when dielectric breakdown occurs and a current flows, the resistance offered between the electrodes is so large that the flowing current is limited.

When an electric waveform of the intermittent current superimposed on a high voltage electric field generated by the electrotherapeutic device in accordance with the present invention is applied to a living body via director electrode plates, reducing ions are generated in that living body. This facilitates alkalization or reduction in a body, and assists vigorously treating deceased parts. Thus, the electrotherapeutic device can facilitate treatment and help to improve fitness.

Traditionally, a higher negative potential applied by an electrotherapeutic device has been thought to be better, and a potential of about - 9 kV has been applied. When the disclosed waveform shaper is employed, the range of the potential values decreases down to about - 3 kV, within which cluster ions formed by electrons released from the electrodes become highly reductive. From this viewpoint, the electrotherapeutic device is quite easy to handle.

When the electrotherapeutic device is applied to a living body, it should preferably be less invasive thereto. Therefore, the director electrode plates to be brought into contact with a human body should adopt a structure having an insulating rubber and a conducting rubber layered on a metallic electrode plate. A current of 10 µA at most can flow so as to guarantee the safety of the human body.

The electrotherapeutic device, the inventors of the present invention have already disclosed, generates a large number of reducing ions in body fluids so as to facilitate activation of immunocompetent cells. When the electrotherapeutic device is applied to a patient infected with a chronic viral infection disease, the patient's immunity is activated to reduce the number of intracorporeal viral particles. Consequently, the symptom is alleviated. When the electrotherapeutic device is applied to a patient infected with a human immunodeficiency virus (HIV) chronic infection disease, the patient's immunity is activated to reduce the number of intracorporeal HIV particles.

The present inventors have developed more effective treatment methods for stimulating immune reaction on infectious diseases using the disclosed electrotherapeutic device.

In the process of the development, the relationships between a treatment current fed by the electrotherapeutic device and indices indicating improvement in immunity have been investigated. Consequently, a specific range of the treatment current values permitting improvement in immunity has been discovered.

The electrotherapeutic device in accordance with the present invention comprises a program controller that controls a treatment current so that the treatment current will fall within the specific range of the treatment current values. The electrotherapeutic device can autonomously optimize the conditions for the treatment according to a patient, an environment, and an environmental change occurring during the treatment process without being handled by an operator. The effect of improving immunity is further intensified.

The electrotherapeutic device in accordance with the present invention intends to utilize an immune reaction mechanism for attacking tumor cells or virus infected cells. The immune mechanism has been clarified to a considerable extent.

The immune mechanism is a system for discovering and eliminating molecules or pathogens that are recognized as being foreign to itself. The immune mechanism does not react on the tissues of a living body. For example, many immuno-therapies against cancer attempt to recognize a cancerous tissue as being more foreign than a normal tissue. Relatively feeble immune reaction on growth of cancer is intensified.

A protecting mechanism for cancer will be described below. Namely, an antigen-presenting cell (APC) picks up a cancer antigenic protein appearing on the surface of a cell membrane and processes it in the APC, and the resultant cancer antigenic protein appears on the surface of the cell together with a major histocompatibility complex (MHC) of class II. An antitumor CD4+T precursor that is a lymphocyte recognizes that cancer antigenic peptide. The CD4+T precursor is led to a helper T cell 1 (Th1) by interleukin-12 (IL-12) released from the antigen-presenting cell (APC). IL-2 and other cytokine released from the activated Th1 activate a natural-killer (NK) cell and a lymphokine activated killer (LAK) cell. A cytotoxic T lymphocyte (CTL) is activated because of coexistence of the activated cells and the cancer antigenic protein, and releases perforin, NO, and FasL. This causes a cancer cell to die.

Macrophages, LAK cells, NK cells, and CTL cells directly attack tumor cells or virus infected cells. Therefore, when the number of those cells is larger and they are more active, the immunity can be higher.

Consequently, a treatment effect can be quantitatively assessed by measuring the activeness of the above cells and others. However, the activeness and the treatment effect cannot be directly measured.

The number of lymphocytes, the number of CD4T lymphocytes, the number of subsets of NK cells, the activity of NK cells, and the number of cytotoxic T lymphocyte cells (CTLs), which can be quantitatively and directly analyzed using the subject's blood, are adopted as indices in order to measure the treatment effect of the electrotherapeutic device.

When the electrotherapeutic device is applied to a subject, a part of a subject's body is sandwiched between a counter electrode plate connected to a ground conductor in a direct current high negative voltage generator and a director electrode plate connected to an output terminal of a waveform shaper. A high voltage superimposed with ripples of sawtooth waveform which are formed by the waveform shaper, is applied to the subject.

The high voltage potential is about DC -6 kV. The director electrode plate is a metallic plate coated with vinyl chloride and is thus insulated. Therefore, only an alternating current component of the treatment current permeates through the subject's body.

The counter electrode plate opposed to the director electrode plate with a body portion between them is connected to the ground electrode in the direct current high negative voltage generator via a protective resistor. The protective resistor prevents an accident that a large current unexpectedly flows into a human body along with application of a high voltage.

In the electrotherapeutic device according to the present invention, a conductor extending from the counter electrode plate is provided with a current sensor that measures a treatment current flowing into a human body. For example, assuming that a resistor offering 100 kΩ is adopted as the current sensor, a voltage of 1 V is developed with the treatment current of 10 µA. This is convenient in transfer of data into a computer and in conversion of the data. A current may be calculated from a voltage measured between the terminals of the resistor coupled to the conductor.

A degree of improvement in cellular immune function achieved by the electrotherapeutic device has been checked and recognized to vary depending on the seasons. For example, the immune function hardly improves in winter and summer but easily improves in spring and autumn. When the electrotherapeutic device is operated under the same conditions, a treatment current decreases in winter but increases in summer, and remains intermediate in spring and autumn.

Many applied cases have been reviewed in terms of the treatment current. Consequently, a larger treatment current does not always lead to a higher degree to which the immune reaction is stimulated. It is estimated that an appropriate range of the current values exists.

The present inventors have investigated the relationships between a treatment current and four indices of the immune reaction, that is, the number of lymphocytes, the number of subsets of NK cells, the activity of NK cells, and the number of cytotoxic T cells (CTLs).

During the treatment, a subject's temperature rises or the director electrode plate is moistened with sweat. Therefore, a treatment current tends to gradually increase. The increase in the treatment current is markedly large in summer.

In experiments of the treatment, an acclimatization time during which immunization is not expected is set at the beginning of the treatment period. When the acclimatization time has elapsed, the treatment current is measured. If the treatment current is excessive, the voltage is regulated to gradually drop. And, when the treatment current is too small e.g. in winter, the voltage is raised gradually in the middle of the treatment for fear the treatment current may become insufficient.

The relationships between the treatment current and immune indices have been checked using a current value attained when the treatment period of twenty min has passed.

For example, the disclosed electrotherapeutic device was applied to a considerable number of sound persons, and the relationship was investigated between a final treatment current and a difference of index values measured before and after the treatment. In this case, when the treatment current attained at the end of the treatment ranges from 1.5 µA to 5.5 µA, the indices are often upgraded after the treatment. As for three indices, that is, the number of lymphocytes, the activity of NK cells, and the number of cytotoxic T cells, when the treatment current ranges from 2.5 µA to 3.8 µA, the indices are largely upgraded. As for the number of subsets of NK cells, when the treatment current ranges both from 2.5 µA to 3.0 µA and from 3.5 µA to 5.5 µA, the index is upgraded in particular.

However, when the treatment current is held nearly constant during the treatment, the above immune indices are not upgraded markedly.

The above results of the experiment demonstrate that the treatment current should be confined to a certain appropriate range and be appropriately varied but not held constant, for an excellent treatment effect.

In the electrotherapeutic device according to the present invention, the program controller applies a predetermined certain voltage from the start of the treatment according to the results of measurement of the treatment current. Once a predetermined time elapses, if the treatment current falls outside a predetermined range of the control current values the program controller regulates the input voltage of the waveform shaper so that the treatment current will gradually approach the range of the control current values. After the treatment current reaches the range of the control current values, the program controller regulates the input voltage of the waveform shaper so that the treatment current will gradually vary within the range of the control current values and finally reach the target current value. Thus, a program for regulating an output voltage during the treatment is implemented.

Consequently, using the electrotherapeutic device in accordance with the present invention, even when an operator does not handle the device during the treatment, a current exhibiting the most preferable pattern is automatically applied to a patient.

Preferably, the range of the control current values is from 1.5 µA to 4.5 µA. The range of the target current values within which the treatment current finally reaches is from 2.0 µA to 4.0 µA, or more precisely, from 2.5 µA to 3.8 µA.

Moreover, the input voltage of the waveform shaper is regulated by regulating the input voltage of the direct current high negative voltage generator that feeds a high voltage electricity to the waveform shaper. The input voltage of the waveform shaper is high, e.g. DC -6 kV. The direct current high negative voltage generator that generates the high voltage electricity receives a low voltage electricity, e.g. DC 12 V, which is easy to treat, steps it up, and then transmits it.

Consequently, it is very easy for a system to regulate the input voltage of the direct current high negative voltage generator according to the results of arithmetic operations performed by a computer. For example, a digital output of the computer is converted into an analog signal, and the input voltage of the direct current high negative voltage generator is varied step by step in units of 10 mV. In this case, the output of DC - 6 kV of the waveform shaper varies step by step in units of 105 V.

The voltage regulation can be programmed so that the voltage will be raised or dropped step by step at intervals of 5 sec, for example.

The treatment current is regulated so that it will range 2 µA to 4 µA at the end of the treatment irrespective of the temperature and so on. Consequently, a difference is minimized in immunity derived from a change of seasons or the temperature.

The electrotherapeutic device according to the present invention is preferably applied with a negative potential to a patient through his or her palm or palms which are sandwiched between the director electrode plate that is a negative electrode and the counter electrode plate. The palm has a small thickness and a dense bloodstream. A distance between the electrode plates is short, and the electric field is strong. The electric field acts greatly on a large amount of blood and takes a great effect. Moreover, since the palm is always exposed to outside, the director electrode plate can be readily applied to the palm. A load on a patient or an operator is limited.

Furthermore, preferably, another counter electrode plate may be added and attached to an instep for the treatment. When the counter electrode plates are attached to a hand and a foot respectively, immunity improves. Experiments demonstrate that immunity is stimulated, though the mechanism of improvement is unknown.

The electrotherapeutic device in accordance with the present invention, for being adapted to such usage, may comprise a counter electrode plate cable to be connected to the counter electrode plate output terminal forming two leads paired with the director electrode plate that is a negative electrode, one lead provided with a counter electrode plate pad at the end to be attached to a palm and another lead provided with another counter electrode plate pad at the end to be attached to an instep.

### [Advantages]

Using the electrotherapeutic device in accordance with the present invention, compared with using a conventional electrotherapeutic device, the density of intracorporeal reducing ions is greatly increased. Active oxygen is eliminated, blood is held alkaline, rouleaux formation of red blood cells is prevented, and bloodstream is stimulated. Consequently, a natural healing power is upgraded, and the treatment effect is improved relative to various diseases.

In particular, the electrotherapeutic device in accordance with the present invention is expected to effectively act on human immunodeficiency virus infection diseases, of which the side effects by chemotherapy have been controversial.

### BRIEF DESCRIPTION OF THE DRAWINGS

Fig. 1 is a block diagram showing an electrotherapeutic device in accordance with an embodiment of the present invention.
Fig. 2 is a cross-sectional view of a waveform shaper employed in the electrotherapeutic device in accordance with the embodiment.
Fig. 3 is a waveform diagram showing an example of an output waveform produced in the electrotherapeutic device in accordance with the embodiment.
Fig. 4 is an explanatory diagram concerning the application of the electrotherapeutic device in accordance with the embodiment to a patient.
Fig. 5 is a distribution chart indicating the relationship between a treatment current and a rate of change in the number of lymphocytes, which is obtained by performing an experiment using the electrotherapeutic device in accordance with the embodiment.
Fig. 6 is a distribution chart indicating the relationship between a treatment current and a rate of change in the number of subsets of NK cells, which is obtained by performing an experiment using the electrotherapeutic device in accordance with the embodiment.
Fig. 7 is a distribution chart indicating the relationship between a treatment current and a rate of change in the activity of NK cells, which is obtained by performing an experiment using the electrotherapeutic device in accordance with the embodiment.
Fig. 8 is a distribution chart indicating the relationship between a treatment current and a rate of change in the number of cytotoxic T cells (CTLs), which is obtained by performing an experiment using the electrotherapeutic device in accordance with the embodiment.
Fig. 9 is a control logic flowchart of a program controller in the electrotherapeutic device in accordance with the embodiment.
Fig. 10 is a graph illustratively indicating a time-sequential change in a treatment current occurring in the electrotherapeutic device in accordance with the embodiment during a treatment time.
Fig. 11 is a graph that compares the rate of change in the number of lymphocytes occurring when another counter electrode plate is added and attached to an instep, with the rate of change therein occurring when the counter electrode plate is attached to a palm alone.
Fig. 12 is a graph that compares the rate of change in the number of subsets of NK cells occurring when another counter electrode plate is added and attached to an instep, with the rate of change therein occurring when the counter electrode plate is attached to a palm alone.
Fig. 13 is a graph that compares the rate of change in the activity of NK cells occurring when another counter electrode plate is added and attached to an instep, with the rate of change therein occurring when the counter electrode plate is attached to a palm alone.
Fig. 14 is a graph that compares the rate of change in the number of cytotoxic T cells (CTLs) occurring when another counter electrode plate is added and attached to an instep, with the rate of change therein occurring when the counter electrode plate is attached to a palm alone.
Fig. 15 is a graph that compares the rate of change in the number of CD4 T lymphocytes occurring another counter electrode plate is added and attached to an instep, with the rate of change therein occurring when the counter electrode plate is attached to a palm alone.

### [Reference Numerals]

- 1:: waveform shaper
- 2:: direct current high negative voltage generator
- 3:: direct current power source
- 4:: relay
- 5:: sequence controller
- 6:: current sensor
- 7:: protective resistor
- 8:: program controller
- 9:: director electrode plate
- 10:: counter electrode plate
- 11:: counter electrode plate
- 12:: subject
- 20:: ground terminal
- 31:: casing
- 32:: inner lid
- 33:: inorganic insulator powder
- 35, 36:: electrode
- 37:: adhesive
- 38,: 39: lead

### DETAILED DESCRIPTION OF THE INVENTION

The present inventors have discovered, as already disclosed, that an electrotherapeutic device using a special waveform shaper filled with pumice blocks or inorganic insulator powder and a predetermined amount of moisture improves the efficiency in generating negative ions or reducing ions and intensifies human immunity. The present inventors have provided an electrotherapeutic device in accordance with the present invention, which will provide a reliable treatment effect and be easy to operate, through profound studies.

The electrotherapeutic device in accordance with the present invention will be described more fully hereinafter in conjunction with an embodiment.

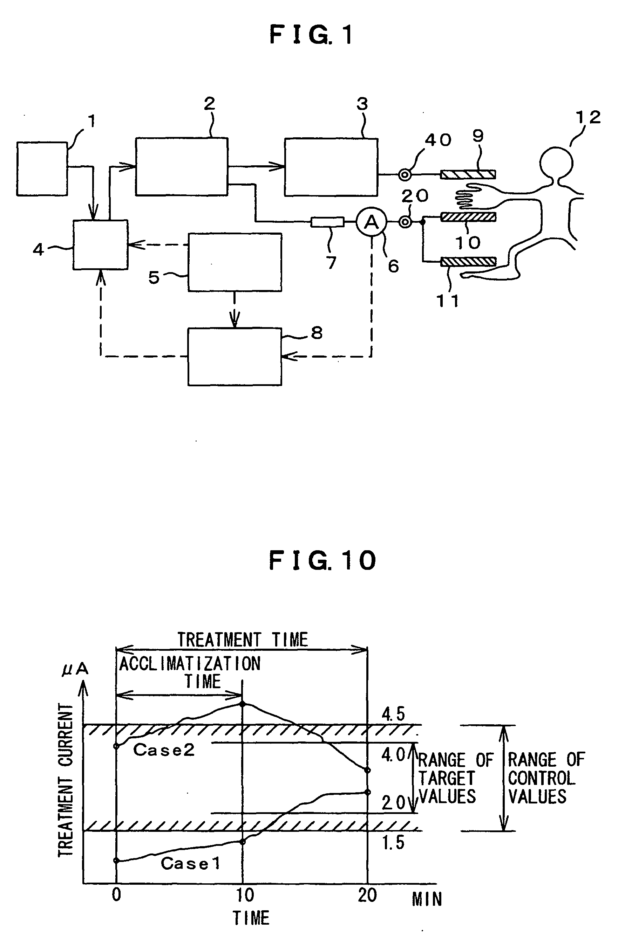

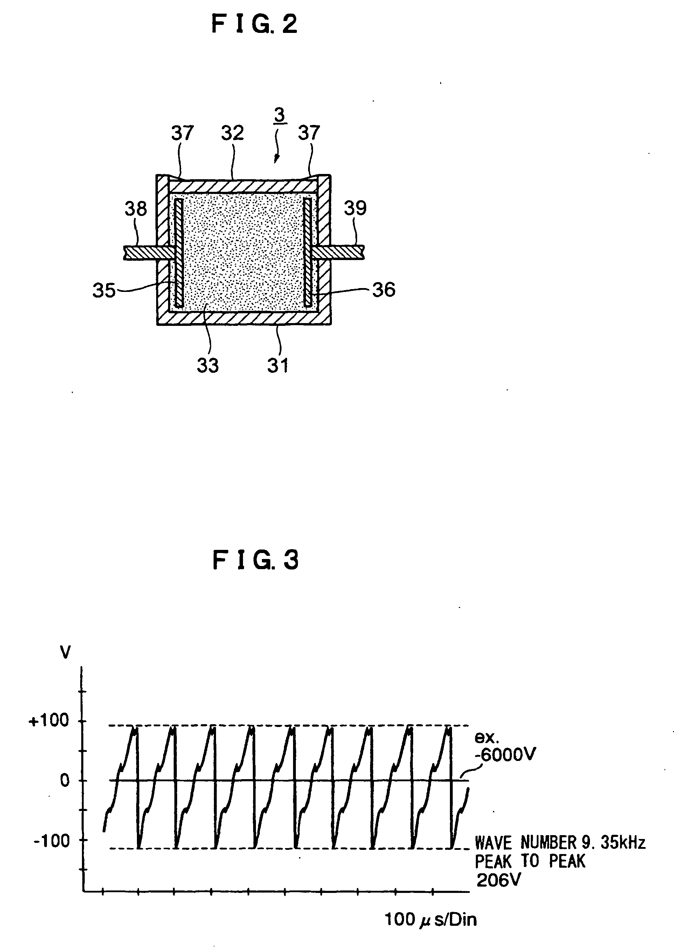

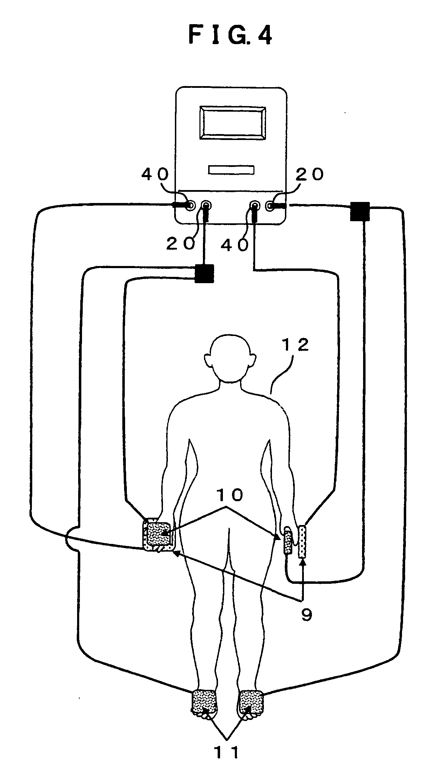

Fig. 1 is a block diagram of the electrotherapeutic device in accordance with an embodiment of the present invention. Fig. 2 is a cross-sectional view of a waveform shaper employed in the present invention. Fig. 3 is an output waveform diagram produced by the present embodiment. Fig. 4 is an explanatory diagram concerning a state in which electrodes are attached to a patient during the treatment.

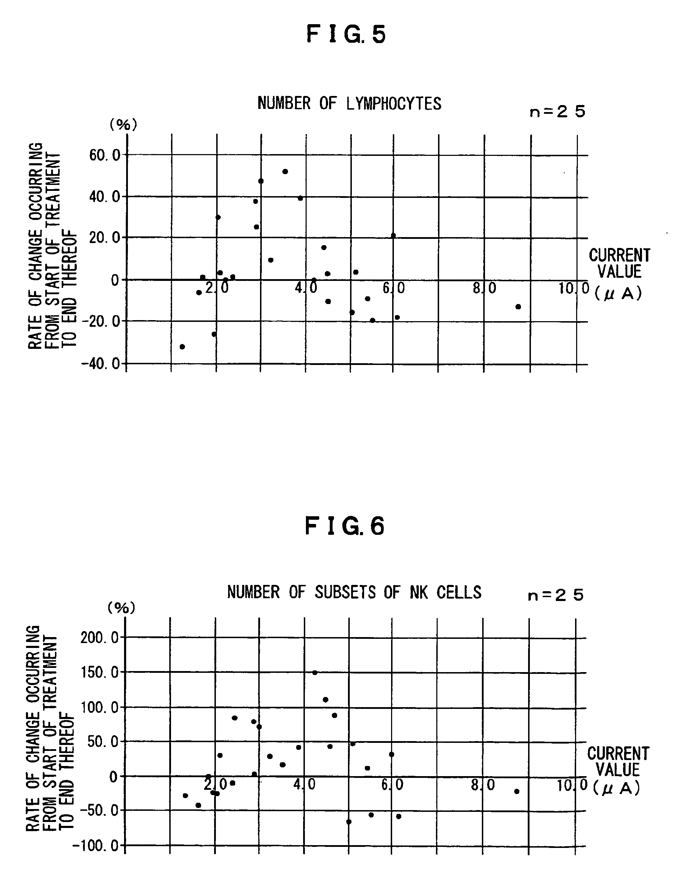

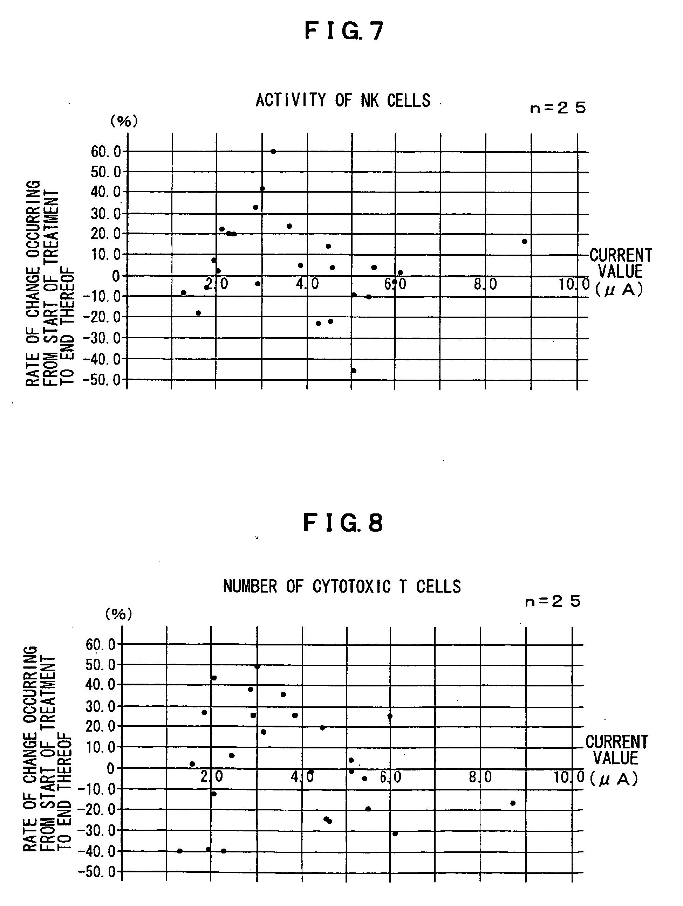

Fig. 5 to Fig. 8 are distribution charts indicating the relationship between a treatment current and a treatment effect, which are obtained by performing an experiment using the electrotherapeutic device in accordance with the present embodiment. Fig. 5 indicates the rate of change in the number of lymphocytes occurring from the start of the experiment to the end thereof plotted in relation to the treatment current values. Fig. 6 indicates the rate of change in the number of subsets of NK cells in relation thereto. Fig. 7 indicates the rate of change in the activity of NK cells in relation thereto. Fig. 8 indicates the rate of change in the number of cytotoxic T cells (CTLs) in relation thereto.

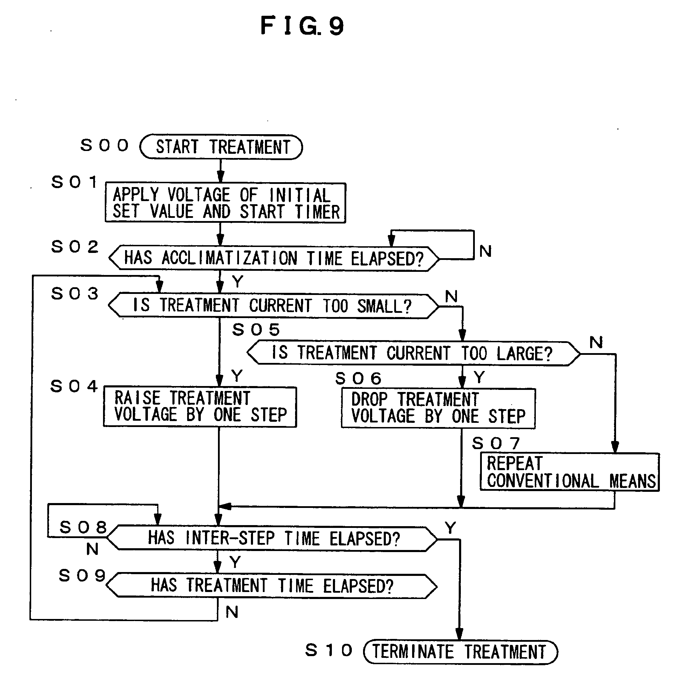

Fig. 9 is a logical flowchart describing an example of control steps to be executed by the program controller 8. Fig. 10 is a graph illustratively indicating the change in a treatment current occurring during a treatment time.

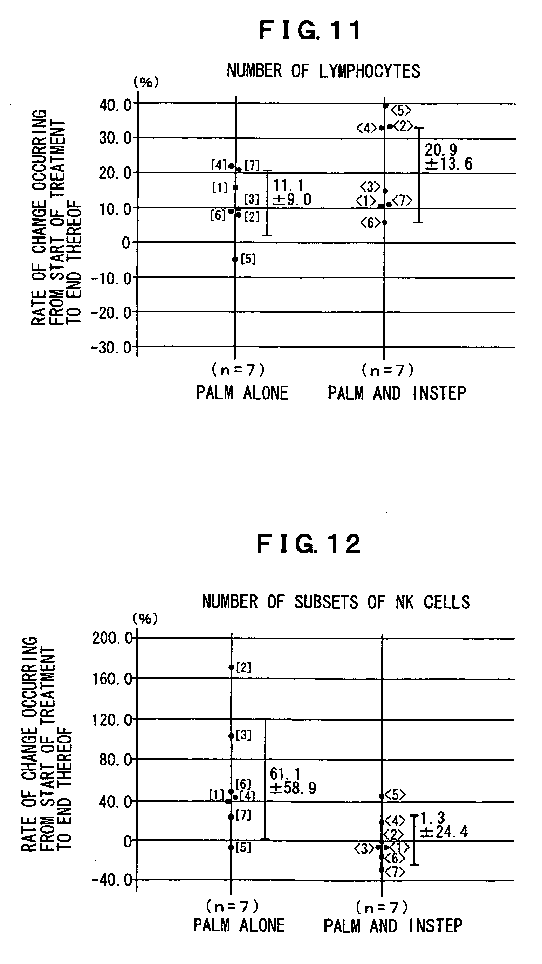

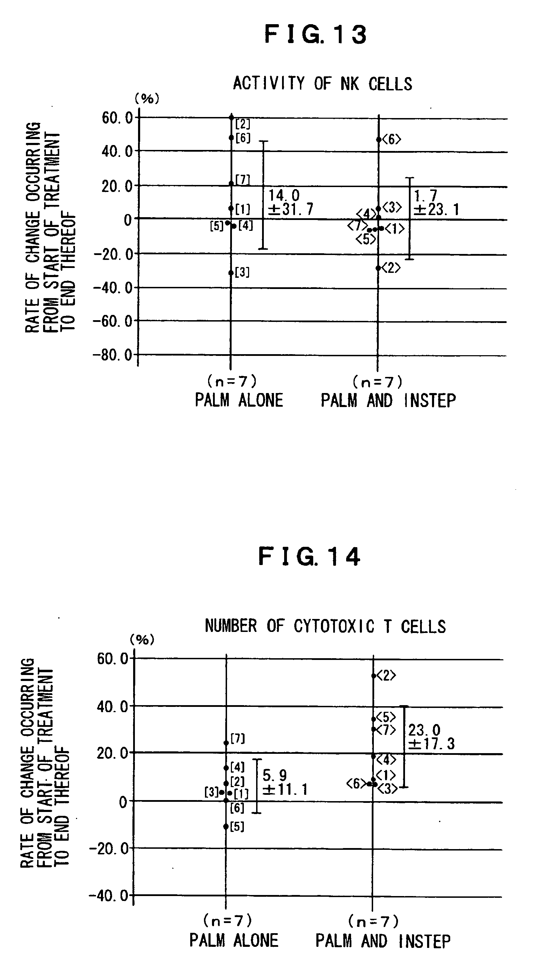

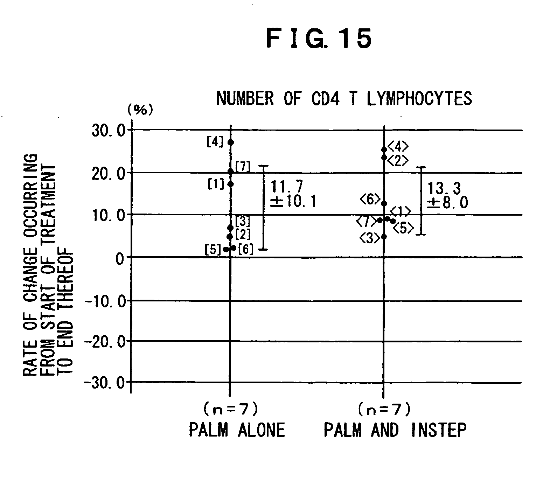

Fig. 11 to Fig. 15 are graphs that compare the rate of change in the number of lymphocytes, the number of subsets of NK cells, the activity of NK cells, the number of cytotoxic T cells (CTLs), and the number of CD4 T lymphocytes, respectively, occurring when another counter electrode plate is added and attached to an instep, with the rate of change therein occurring when the counter electrode plate is attached to a palm alone.

The electrotherapeutic device in accordance with the present embodiment includes as major components a direct current power source 1, a direct current high negative voltage generator 2, a waveform shaper 3, a relay 4, a sequence controller 5, a current sensor 6, a protective resistor 7, and a program controller 8. A director electrode plate 9, and counter electrode plates 10 and 11 are connected to the electrotherapeutic device via terminals, and attached to a patient. A greater treatment effect is expected when the electrode plates are attached to the patient's left hand and left foot and the patient's right hand and right foot at the same time. Therefore, an electrotherapeutic device including two sets of electrode units may be used to attach the electrode plates to both the right hand and foot and the left hand and foot, simultaneously.

The direct current power source 1 is an AC-DC converter that converts an AC 100 V into a DC 12 V. A 0 V output terminal is grounded.

The direct current high negative voltage generator 2 is realized with a booster that includes electromagnetic coils and rectifiers. A high-frequency chopping circuit produces an alternating current voltage from a direct current voltage of 12 V supplied from the direct current power source 1 via the relay 4. The generator steps up the alternating current voltage by the coils and fully rectify the current. The voltage is smoothed by a capacitor. Consequently, a high voltage direct current of - 9 kV at most is transmitted via an output terminal.

The output voltage can be regulated by the supplied direct current voltage. For example, assuming that the input voltage is varied by 10 mV, the output voltage varies by 105 V.

The relay 4 controls connection and disconnection of input voltage to the direct current high negative voltage generator 2 according to a signal sent from the sequence controller 5.

The high negative voltage terminal of the direct current high negative voltage generator 2 is connected to the input terminal of the waveform shaper 3. The waveform shaper 3 is a capacitive distributed constant circuit element offering a high resistance, which shapes up a high direct current voltage signal so that a current will flow intermittently.

The waveform shaper 3 is, for example as shown in Fig. 2, formed by filling an airtight space, which is defined by a non-conducting casing 31 made of a synthetic resin and an inner lid 32, with porous inorganic insulator powder 33 which contains a predetermined amount of moisture.

A pair of electrode plates 35 and 36 is abutted on the opposite walls of the casing 31 with the inorganic insulator powder 33 between them. One of the electrode plates, that is, the electrode 35 is connected to the high negative voltage terminal of the direct current high negative voltage generator 2 via a lead 38. The other electrode 36 is connected to a director electrode plate output terminal 40 via a lead 39. The inner lid 32 is bonded to the casing 31 using an adhesive, whereby the waveform shaper 3 is kept airtight.

The moistened inorganic insulator is not a full insulator. When the space filled with the inorganic insulator in a close-packed state between the electrodes is applied with a high voltage, a phenomenon similar to dielectric breakdown occurs. Consequently, a current slightly flows. After a voltage drops, an insulated state is restored. The waveform shaper exhibiting such a nonlinear characteristic superimposes a special pulsating waveform on a high negative voltage as shown in Fig. 3.

As for the inorganic insulator powder 33, finely granular pumice blocks may be adopted.

The director electrode plate 9 is connected to the output terminal 40 of the waveform shaper 3. The ground terminal of the direct current high negative voltage generator 2 is connected to a counter electrode plate output terminal 20 via the protective resistor 7 and the current sensor 8. Two counter electrode plates 10 and 11 are connected to the counter electrode plate output terminal 20.

Conventionally, two pairs of a director electrode plate and a counter electrode plate are used to attach to the right and left palms for the treatment. Experiments demonstrate that when another counter electrode plate is also attached to each of the right and left insteps, a treatment effect markedly improves, though the mechanism is unknown. Consequently, the electrotherapeutic device in accordance with the present embodiment adopts a cable terminated with two of parallel-connected counter electrode plate pads.

For the treatment, as shown in Fig. 4, a palm of a subject 12 is interposed between the director electrode plate 9 and the first counter electrode plate 10, and the second counter electrode plate 11 is attached to an instep of the subject 12. The first counter electrode plate 10 and the second counter electrode plate 11 are attached to a hand and a foot of the same side. Normally, two pairs of electrode units are used to attach the electrodes to the right and left hands and feet respectively.

After the electrodes are attached, a treatment time is set, and a start switch of the electrotherapeutic device is turned on. Consequently, a special waveform output produced by the waveform shaper 3 is applied to the subject and the treatment is initiated. The treatment time can be selected in units of 1 min and is normally set to about 20 min.

The conditions of dryness of a subject's skin, the insulation resistance of electrode pads, and so on, vary depending on the nature or condition of the individual subject, and the climate or weather such as the temperature or humidity. Even if an applied electric waveform remains unchanged, a current flowing through a subject's body varies every time.

Investigation on a degree of improvement in cellular immune function achieved by the electrotherapeutic device demonstrates that the immune function improves less in winter and summer but improves more in spring and autumn. Even when the same voltage is applied, a treatment current decreases in winter but increases in summer and remains intermediate in spring and autumn.

Moreover, a subject's temperature rises or a body portion to which an electrode pad is attached is sweated during the treatment. Normally, as the treatment progresses, the electric resistance tends to decrease and the treatment current increase.

An output of an appropriate initial applied voltage is set and applied in order to commence the treatment. Generally, a treatment current gradually increases. However, unless danger is foreseen, the treatment current is kept increasing until an appropriate time elapses. The time may be set to about ten min and be considered as an acclimatization time required for a patient's body to get acclimatized to the treatment current.

When the treatment current is too small, no treatment effect is exerted. However, a large treatment current does not always lead to an excellent treatment effect. An appropriate range of the treatment current values exists.

An experiment was conducted on twenty-five sound persons, who are subjects, in order to detect a range of the treatment current values bringing about an excellent treatment effect. Fig. 5 to Fig. 8 are distribution charts indicating the relationship between a treatment current and a treatment effect, which are obtained by performing the experiment. Fig. 5 indicates the rate of change in the number of lymphocytes occurred between the start of the experiment and the end thereof plotted in relation to the treatment current values. Fig. 6 indicates the rate of change in the number of subsets of NK cells in relation thereto. Fig. 7 indicates the rate of change in the activity of NK cells in relation thereto. Fig. 8 indicates the rate of change in the number of cytotoxic T cells (CTLs) in relation thereto.

Herein, the treatment current value is substituted by a value detected at the end of the treatment time.

From the results of the experiment, although the relationship between the treatment current and the improvement in immunity varies greatly, it can be understood that the indices are improved in a range from 1.5 µA to 4.5 µA of the treatment current detected at the end of the treatment. The distributions of values of the rate of change in the number of lymphocytes, the number of subsets of NK cells, and the activity of NK cells exhibit the same tendency. The rate of change has a peak around the treatment current value of 3 µA. When the treatment current exceeds 6 µA, the improvement in the treatment effect is not observed. The positive values of the rate of change are distributed in a range of the treatment current values from 2 µA to 4 µA. In particular, when the treatment current ranges from 2.5 µA to 3.8 µA, the indices are observed much improved. Moreover, when the treatment current ranges from 2.5 µA to 3 µA and from 3.5 µA to 5.5 µA, the rate of change in the number of cytotoxic T cells (CTLs) has a peak.

Assuming that the treatment current is held nearly constant during the treatment, the immune indices are not improved markedly.

Consequently, for a good effect of treatment, the treatment current should be confined within an appropriate range of the control current values, e.g. from 2 µA to 4 µA. Moreover, the treatment current should not be held constant but should be varied appropriately.

In the electrotherapeutic device according to the present embodiment, the range of the control current values is set from 1.5 µA to 4.5 µA, and the range of the target current values within which the treatment current should reach finally in the treatment is set from 2.0 µA to 4.0 µA, or more precisely, from 2.5 µA to 3.8 µA. Needless to say, the other range of current values may be selected for adapting to other conditions.

The range of the target current values is relatively fixed but is not varied depending on a season and so on. And also, the range of the target current values is not affected by patient's conditions. The same values can be utilized as the target current values. Consequently, set values may be written in a program in advance for fear an operator may easily change the values.

During the treatment time, the program controller 8 autonomously controls the treatment current. The contents of the control will be described below in conjunction with the logical flowchart of Fig. 9 and the graph of Fig. 10.

When the initiation of the treatment is instructed (S00), an initial applied voltage of, e.g. - 6 kV, is applied to a subject, and a built-in timer is started. The electrotherapeutic device is left intact for the acclimatization time of, e.g. ten min (S01). After the acclimatization time elapses, the treatment current is checked to see if it falls outside the range of the control current values (S02). The treatment current is detected by the current sensor 6 and the measured value is transferred to the program controller 8.

The treatment current is compared with the lower limit of the range of the control current values (S03). If the treatment current falls below the lower limit similarly to case 1 shown in Fig. 10, the applied voltage is raised by one step, e.g. 105 V (S04). If the treatment current exceeds the upper limit similarly to case 2 shown in Fig. 10 (S05), the applied voltage is dropped by one step (S06).

Incidentally, when the treatment current falls within the range of the control current values, the applied voltage is treated repeatedly as it is previously (S07). Namely, if the applied voltage is raised by one step previously, it is raised by one step. If the applied voltage is dropped by one step previously, it is dropped by one step.

The applied voltage is regulated by controlling a direct current voltage provided to the direct current high negative voltage generator 2 serving as a high voltage power source for the waveform shaper 3. A result of computation performed by the program controller 8 is converted into an analog form, and the resultant voltage is supplied to a 12 V input terminal of the direct current high negative voltage generator 2. For example, when the input voltage of the direct current high negative voltage generator 2 is varied by 10 mV, the output voltage varies by 105 V.

Determinations are made on whether the treatment current exceeds or falls below the range of the control current values with every elapse of a certain predetermined time of, e.g. 5 sec (S08), until the initially set treatment time of, e.g. 20 min, elapses (S09). The applied voltage is thus regulated (S03 to S07).

Incidentally, if the treatment current is about to fall outside the range of the control current values, the treatment current may be controlled to fall within the range of the control current values according to the same logic.

Owing to the operation of the program controller 8, during the treatment time succeeding the acclimatization time, while the applied voltage is gradually varied, the treatment current can be slowly changed to fall within the range of the target current values finally.

The treatment current most greatly stimulating immunity is possibly different depending on the immune mechanism. Therefore, the treatment current may be varied so that it goes up and down within the range of the target current values. For this purpose, the voltage step by which the applied voltage is changed is increased or the interval between the steps is shortened so that the treatment current will go up and down to reach the lower and the upper limits of the range of the control current values.

The electrotherapeutic device in accordance with the present embodiment comprises the program controller. The program controller autonomously controls the treatment current. After the treatment current reaches the range of the control current values, the program controller regulates the input voltage of the waveform shaper so that the treatment current will gradually vary within the range of the control current values and finally reach the target current value. Consequently, even when an operator does not handle the device during the treatment, the most preferable pattern of the treatment current is automatically applied to the patient.

As mentioned above, the input voltage of the waveform shaper is regulated so that the treatment current will be confined to the range of the target current values at the end of the treatment irrespective of the temperature, humidity, or patient's temperature. Consequently, the immunity can be properly improved.

The electrotherapeutic device in accordance with the present embodiment comprises a counter electrode plate cable which is diverged into two leads from a lead connected to the counter electrode plate output terminal, and which is provided with the first counter electrode plate pad 10 to be attached to a palm at the end of the first lead and the second counter electrode plate pad 11 to be attached to an instep at the end of the second lead.

For applying the electrotherapeutic device according to the present embodiment, a palm of the patient 12 is sandwiched between the director electrode plate 9 that is negative electrode and the first counter electrode plate 10 as conventional. A negative potential having ripples of the special waveform superimposed thereon is applied to the electrodes for use.

In the electrotherapeutic device according to the present embodiment, preferably, the second counter electrode plate 11 may also be attached to an instep for the treatment.

When the counter electrode plates are attached to a hand and a foot respectively, it is observed in experiments that the immunity is improved, though the mechanism of improvement is unknown.

For example, when the counter electrode plates were attached to a hand and a foot respectively of each of two patients suffering from the type C chronic hepatitis, the number of viral particles of the type C hepatitis was reduced in the two patients. At this time, all of the cellular immunity was improved, except the activity of NK cells.

In the case of a patient having interferon administered thereto, the number of cytotoxic T cells was improved more apparently than the other indices of cellular immunity. For suppression of a type C hepatitis virus, increasing the number of cytotoxic T cells is thought to be more significant than stimulation of the activity of NK cells. Consequently, when the diverged counter electrode plate is additionally attached to an instep, a treatment effect on a patient suffering from type C chronic hepatitis is expected.

The present inventors conducted an experiment to check the effect of the second counter electrode plate 11 attached to a foot. In the experiment, at first, the palm was sandwiched between the director electrode plate 9 and the first counter electrode plate of the electrotherapeutic device, for the treatment. The rates of change in the number of lymphocytes, the number of subsets of NK cells, the activity of NK cells, the number of cytotoxic T cells (CTLs), and the number of CDE4 T lymphocytes occurring from the start of the treatment to the end thereof were measured. In addition, the rates of change in the above indices were measured with the second counter electrode plate additionally attached to the instep. The results of measurements in both cases were compared with each other.

Seven sound persons were selected as subjects and measured for each case.

Fig. 11 to Fig. 15 are graphs that compare the rate of change in the number of lymphocytes, the number of subsets of NK cells, the activity of NK cells, the number of cytotoxic T cells (CTLs), and the number of CD4 T lymphocytes, respectively, occurring when the counter electrode plates included in the electrotherapeutic device in accordance with the present embodiment are attached not only to a palm but also to an instep, with the rate of change therein occurring when the counter electrode plate is attached to a palm alone.

Among the group of subjects, the improvement of the cellular immunity could be seen apparently as follows. For the subjects each having the counter electrode plate attached to the palm alone, the three indices, that is, the number of lymphocytes, the number of subsets of NK cells, and the number of CD4 T lymphocytes were improved.

For the subjects each having the counter electrode plate attached also to the instep, the three indices, that is, the number of lymphocytes, the number of cytotoxic T cells (CTLs), and the number of CD4 T lymphocytes were improved.

The rate of change in two indices, that is, the number of lymphocytes, and the number of cytotoxic T cells (CTLs) was understood to be larger for the subjects having the counter electrode plates attached to a palm and an instep respectively than for the subjects having the counter electrode plate attached to a palm alone.

Consequently, it was confirmed that applying the diverged counter electrode plates attached to a palm and an instep respectively would be effective for the patient suffering from the type C chronic hepatitis, e.g., to whom the increasing of the number of cytotoxic T cells (CTLs) would presumably be effective.

For comparison, the immune indices of other group of subjects were measured by sandwiching a palm between the director electrode plate and the counter electrode plate and grounding another counter electrode plate attached to an instep, and by sandwiching only an instep between the director electrode plate 9 and the first counter electrode plate 10.

When the instep was sandwiched between the director electrode plate and the counter electrode plate, any of the indices were not statistically significantly improved. And, when the counter electrode plate attached to the instep was grounded, the activity of NK cells alone was significantly improved but the other indices were not significantly improved.

Consequently, grounding the counter electrode plate attached to an instep did not prove effective. Moreover, attaching the counter electrode plate to an instep did not prove effective. It was confirmed that applying the director electrode plate 9 and the first counter electrode plate 10 attached to a palm and also applying the second counter electrode plate connected in parallel with the first counter electrode plate attached to an instep would be effective for the improvement of the cellular immunity.

The electrotherapeutic device according to the present invention comprising the waveform shaper regulates a treatment current to fall within a specific range of the current values without the operator's labor. Thereby reducing ions or an effect of reducing ions is generated in a living body. The number of intracorporeal immunocompetent cells is markedly increased. The large number of immunocompetent cells attacks virus-infected cells so as to eliminate or decrease the number of viral particles. Consequently, the electrotherapeutic device is effective in treating a virus-infected patient whose immunity is decreased.

## Claims

1. An electrotherapeutic device comprising a waveform shaper (3) filled with pumice blocks or inorganic insulator powder (33) and a specific amount of moisture arranged in series between a direct current high negative voltage generator (2) and a director electrode plate output terminal (40), and an output voltage fed from the direct current high negative voltage generator (2) is supplied to the director electrode plate output terminal (40) via the waveform shaper (3), **characterized by** comprising:
a current sensor (6) for measuring a treatment current; and
a program controller (8) for varying an output voltage fed from the director electrode plate output terminal (40), according to a predetermined program, wherein:
the program controller (8) applies a certain predetermined voltage until a predetermined acclimatization time has elapsed from the start of the treatment;
once the acclimatization time has elapsed, if the treatment current falls outside a predetermined range of control current values the program controller (8) regulates the input voltage of the waveform shaper (3) so that the treatment current will gradually approach the range of the control current values;
after the treatment current reaches the range of the control current values, the program controller (8) regulates the input voltage of the waveform shaper (3) so that the treatment current will gradually vary within the range of the control current values; and
the program controller (8) thus regulates the output voltage to be applied during a succeeding treatment period.

2. The electrotherapeutic device according to claim 1, wherein the range of the control current values is from 1.5 µA to 4.5 µA.

3. The electrotherapeutic device according to claim 1, wherein the range of the control current values is from 2.5 µA to 3.8 µA.

4. The electrotherapeutic device according to claim 1, comprising a counter electrode plate cable which is diverged from a lead connected to the counter electrode plate output terminal (20) forming a pair with the director electrode plate output terminal (40), and which is provided with a first counter electrode plate pad (10) to be attached to a palm at one end thereof and a second counter electrode plate pad (11) to be attached to an instep at the other end thereof.

5. The electrotherapeutic device according to claim 1, wherein the input voltage of the waveform shaper (3) is regulated by regulating the input voltage of the direct current high negative voltage generator (2).

6. The electrotherapeutic device according to claim 1, wherein the inorganic insulator powder (33) consists of porous stones whose diameter ranges from 1 µm to 200 µm.

7. The electrotherapeutic device according to claim 6, wherein the waveform shaper (3) includes the amount of moisture, which ranges from 2.5 volume% to 3.5 volume%.

8. The electrotherapeutic device according to claim 1, wherein the voltage ranging from - 3 kV to - 9 kV is applied to the waveform shaper (3).

## Patentansprüche

1. Elektrotherapeutische Vorrichtung, umfassend eine mit Bimsblöcken oder anorganischem Isolierpulver (33) und einer spezifischen Feuchtigkeitsmenge gefüllte Wellenformungseinrichtung (3), angeordnet in Reihe zwischen einem Generator (2) von Gleichstrom mit hoher negativer Spannung und einem Richtelektrodenplatten-Ausgangsanschluss (40), wobei eine Ausgangsspannung, eingespeist aus dem Generator (2) von Gleichstrom mit hoher negativer Spannung, dem Richtelektrodenplatten-Ausgangsanschluss (40) via die Wellenformungseinrichtung (3) zugeführt wird, **gekennzeichnet durch** das Umfassen:
eines Stromsensors (6) zum Messen eines Behandlungsstroms; und
einer Programmsteuerungseinrichtung (8) zum Variieren einer Ausgangsspannung, die vom Richtelektrodenplatten-Ausgangsanschluss (40) zugeführt wird, in Übereinstimmung mit einem zuvor festgelegten Programm, wobei:
die Programmsteuerungseinrichtung (8) eine bestimmte vorgegebene Spannung anlegt, bis eine vorgegebene Gewöhnungszeit seit Beginn der Behandlung verstrichen ist;
sobald die Gewöhnungszeit verstrichen ist, die Programmsteuerungseinrichtung (8), falls der Behandlungsstrom außerhalb eines vorgegebenen Bereichs von Kontrollstromwerten fällt, die Eingangsspannung der Wellenformungseinrichtung (3) regelt, so dass sich der Behandlungsstrom allmählich dem Bereich der Kontrollstromwerte nähert;
nachdem der Behandlungsstrom den Bereich der Kontrollstromwerte erreicht hat, die Programmsteuerungseinrichtung (8) die Eingangsspannung der Wellenformungseinrichtung (3) regelt, so dass der Behandlungsstrom allmählich innerhalb des Bereichs der Kontrollstromwerte variiert; und
die Programmsteuerungseinrichtung (8) somit die Ausgangsspannung regelt, die während eines folgenden Behandlungszeitraums anzuwenden ist.

2. Elektrotherapeutische Vorrichtung nach Anspruch 1, bei welcher der Bereich der Kontrollstromwerte von 1,5 µA bis 4,5 µA geht.

3. Elektrotherapeutische Vorrichtung nach Anspruch 1, bei welcher der Bereich der Kontrollstromwerte von 2,5 µA bis 3,8 µA geht.

4. Elektrotherapeutische Vorrichtung nach Anspruch 1, umfassend ein Gegenelektrodenplattenkabel, welches von einer Leitung abgezweigt ist, die an den Gegenelektrodenplatten-Ausgangsanschluss (20) angeschlossen ist, der ein Paar mit dem Richtelektrodenplatten-Ausgangsanschluss (40) bildet, und welches ausgestattet ist mit einem ersten Gegenelektrodenplattenpad (10), anzubringen an einer Handfläche, an dem einen Ende davon und einem zweiten Gegenelektrodenplattenpad (11), anzubringen an einem Fußrücken, an dem anderen Ende davon.

5. Elektrotherapeutische Vorrichtung nach Anspruch 1, bei welcher die Eingangsspannung der Wellenformungseinrichtung (3) geregelt wird, indem die Eingangsspannung des Generators (2) von Gleichstrom mit hoher negativer Spannung geregelt wird.

6. Elektrotherapeutische Vorrichtung nach Anspruch 1, bei welcher das anorganische Isolierpulver (33) aus porösen Steinchen besteht, deren Durchmesser im Bereich von 1 µm bis 200 µm liegt.

7. Elektrotherapeutische Vorrichtung nach Anspruch 6, bei welcher die Weilenformungseinrichtung (3) eine Feuchtigkeitsmenge enthält, die im Bereich von 2,5 Vol.-% bis 3,5 Vol.-% liegt.

8. Elektrotherapeutische Vorrichtung nach Anspruch 1, bei welcher eine Spannung im Bereich von -3 kV bis -9 kV an die Wellenformungseinrichtung (3) gelegt wird.

## Revendications

1. Dispositif électrothérapeutique comprenant un conformateur de forme d'onde (3), rempli de blocs de pierre ponce ou d'une poudre isolante inorganique (33) et d'une quantité spécifique d'humidité, agencé en série entre un générateur de haute tension négative en courant continu (2) et une borne de sortie d'une plaque d'électrode directrice (40), une tension de sortie fournie par le générateur de haute tension négative en courant continu (2) étant appliquée à la borne de sortie de plaque d'électrode directrice (40) via le conformateur de forme d'onde (3), **caractérisé en ce qu'**il comprend :
un détecteur de courant (6) mesurant un courant de traitement ; et
un contrôleur de programme (8) faisant varier une tension de sortie fournie par la borne de sortie de plaque d'électrode directrice (40), conformément à un programme prédéterminé, dans lequel :
le contrôleur de programme (8) applique une certaine tension prédéterminée pendant un temps d'acclimatation prédéterminé à compter du début du traitement ;
une fois que le temps d'acclimatation s'est écoulé, si le courant de traitement chute hors d'une plage prédéterminée des valeurs de courant de commande, le contrôleur de programme (8) régule la tension d'entrée du conformateur de forme d'onde (3) de telle sorte que le courant de traitement s'approche progressivement de la plage des valeurs de courant de commande ;
après que le courant de traitement a atteint la plage des valeurs de courant de commande, le contrôleur de programme (8) régule la tension d'entrée du conformateur de forme d'onde (3) de telle sorte que le courant de traitement varie progressivement au sein de la plage des valeurs de courant de commande ; et
le contrôleur de programme (8) régule ainsi la tension de sortie devant être appliquée lors d'une période de traitement suivante.

2. Dispositif électrothérapeutique selon la revendication 1, dans lequel la plage des valeurs de courant de commande va de 1,5 µA à 4,5 µA.

3. Dispositif électrothérapeutique selon la revendication 1, dans lequel la plage des valeurs de courant de commande va de 2,5 µA à 3,8 µA.

4. Dispositif électrothérapeutique selon la revendication 1, comprenant un câble de plaque de contre-électrode qui est dérivé d'un fil relié à la borne de sortie de la plaque de contre-électrode (20) constituant une paire avec la borne de sortie de plaque d'électrode directrice (40), et qui est pourvu d'une première pastille de plaque de contre-électrode (10) destinée à être rattachée à une paume à l'une de ses extrémités, et d'une seconde pastille de plaque de contre-électrode (11) destinée à être rattachée à un cou-de-pied à l'autre de ses extrémités.

5. Dispositif électrothérapeutique selon la revendication 1, dans lequel la tension d'entrée du conformateur de forme d'onde (3) est régulée par régulation de la tension d'entrée du générateur de haute tension négative en courant continu (2).

6. Dispositif électrothérapeutique selon la revendication 1, dans lequel la poudre isolante inorganique (33) consiste en des pierres poreuses dont le diamètre va de 1 µm à 200 µm.

7. Dispositif électrothérapeutique selon la revendication 6, dans lequel le conformateur de forme d'onde (3) comporte une quantité d'humidité allant de 2,5 % en volume à 3,5 % en volume.

8. Dispositif électrothérapeutique selon la revendication 1, dans lequel une tension allant de - 3 kV à - 9 kV est appliquée au conformateur de forme d'onde (3).
